# EUROPEAN PATENT APPLICATION

(11) **EP 4 372 009 A1**
(43) Date of publication of application: **22.05.2024**
(21) Application number: 22849465.4
(22) Date of filing: 26.07.2022
(51) Int. Cl.: C07K 16/46, C12N 5/10, C12N 15/13, C12N 15/62, C12N 15/82, G01N 33/531, C12P 21/08

(54) **REAGENT CONTAINING HUMANIZED ANTIBODY**

(30) Priority: 26.07.2021 JP 2021121812
(71) Applicant: Denka Company Limited, Tokyo 103-8338 (JP)
(72) Inventor: ISHIKAWA Haruto, Gosen-shi, Niigata 959-1695 (JP); YAMAMOTO Kimitaka, Gosen-shi, Niigata 959-1695 (JP); IZUTANI Noriyuki, Machida-shi, Tokyo 194-8560 (JP); OGASAWARA Daisuke, Machida-shi, Tokyo 194-8560 (JP); WAKABAYASHI Yuki, Machida-shi, Tokyo 194-8560 (JP)
(74) Representative: Schrell, Andreas
(86) International application number: PCT/JP2022/028698
(87) International publication number: WO 2023/008404

(57) **Abstract**

The invention provides a reagent, a kit for measuring an antigen, and a method for measuring an antigen using an antibody capable of reducing a non-specific reaction. The immunoassay reagent comprises an humanized antibody against a target substance.

## Description

### Technical Field

The present invention relates to a humanized antibody, a fragment thereof (e.g., a variable domain or scFv), a conjugate comprising such humanized antibody or fragment (e.g., fusion protein), and use of such humanized antibody or fragment in diagnostic and therapeutic pretargeting methods and compositions. The present invention further relates to use of such humanized antibody in conventional immunotherapeutic and immunodiagnostic methods and compositions for disease treatment and imaging and for other purposes.

### Background Art

A non-human animal-derived monoclonal antibody has the immunogenic potential against an immune cell or antibody included in a human analyte, which would cause a non-specific reaction, disadvantageously.

Antibodies against non-human animal antibodies existing in human blood are referred to as "heterophilic antibodies." An immunoassay technique often involves the use of mouse-derived monoclonal antibodies. Accordingly, non-specific reactions caused by human antimouse antibodies (HAMA) are often observed in immunoassays. Approximately 10% of human analytes contain HAMA.

In the past, a compound that would inhibit non-specific reactions caused by heterophilic antibodies was added to an assay system (see Patent Literature 1).

### Citation List

### Patent Literature

Patent Literature 1: JP Patent Publication (Kokai) No. 2008-249738 A

### Summary of Invention

### Technical Problem

Under the above circumstances, the present invention provides a reagent, a kit for measuring an antigen, and a method for measuring an antigen using an antibody capable of reducing a non-specific reaction.

### Solution to Problem

The present inventors discovered that use of a humanized antibody would reduce a non-specific antigen-antibody reaction, thereby completing the present invention.

Specifically, the present invention is as described below.
[1] An immunoassay reagent comprising a humanized antibody against a target substance.
[2] The reagent according to [1], wherein the humanized antibody inhibits a non-specific reaction.
[3] The reagent according to [1] or [2], wherein the humanized antibody is IgG.
[4] The reagent according to [1] or [2], wherein the humanized antibody is produced using a plant expression system.
[5] The reagent according to any of [1] to [4], which is used for diagnosis.
[6] The reagent according to any of [1] to [5], which is used for research.
[7] A kit comprising the reagent according to any of [1] to [6].
[8] An immunoassay method comprising bringing a humanized antibody against a target substance into contact with the target substance.
[9] The immunoassay method according to [8], wherein the humanized antibody inhibits a non-specific reaction.
[10] The immunoassay method according to [8] or [9], wherein the humanized antibody is IgG.
[11] The immunoassay method according to [8] or [9], wherein the humanized antibody is produced using a plant expression system.
[12] A method for inhibiting a non-specific antigen-antibody reaction in an immunoassay method involving the use of a humanized antibody against a target substance.
[13] The method for inhibiting a non-specific reaction according to [12], wherein the humanized antibody inhibits a non-specific reaction.
[14] The method for inhibiting a non-specific reaction according to [12] or [13], wherein the humanized antibody is IgG.
[15] The method for inhibiting a non-specific reaction according to [12] or [13], wherein the humanized antibody is produced using a plant expression system.

The description incorporates the contents disclosed by JP Patent Application No. 2021-121812, based on which the priority of the present application claims.

### Advantageous Effects of Invention

The present invention can provide a reagent that can inhibit a non-specific reaction with the use of a humanized antibody.

### Brief Description of Drawings

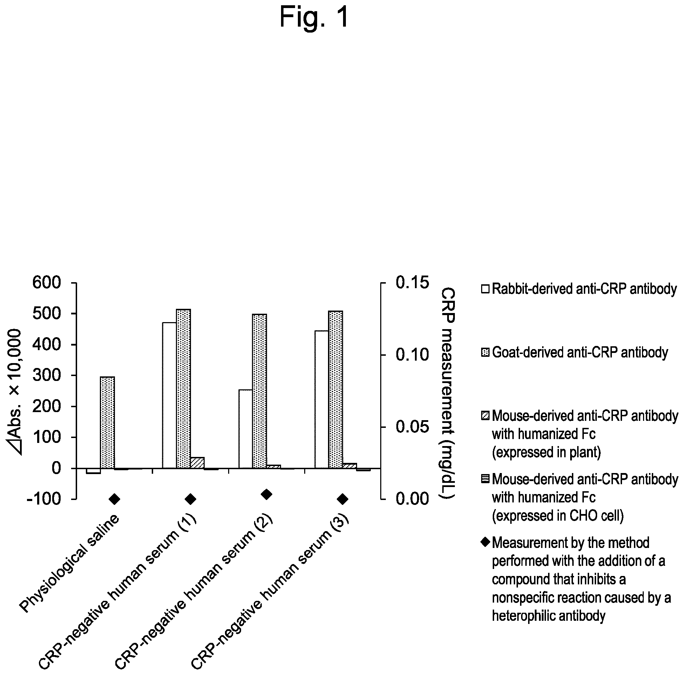

[Figure 1] Figure 1 shows the results of measuring physiological saline and CRP-negative serum analytes using a reagent containing a rabbit-derived anti-CRP antibody, a goat-derived anti-CRP antibody, or a mouse-derived anti-CRP antibody by a method of measurement performed with the addition of a compound that inhibits a non-specific reaction caused by a heterophilic antibody and by a method of measurement performed without the addition of a compound that inhibits a non-specific reaction caused by a heterophilic antibody.

### Description of Embodiments

Hereafter, the present invention is described in detail.

In the present invention, a humanized antibody is used as an antibody binding to a target substance (to be measured) in an antigen-antibody-reaction-based immunoassay method. This can inhibit a non-specific reaction caused by an antibody against a non-human animal antibody produced with the use of a non-human animal-derived antibody.

### (Reagent)

The first aspect of the present invention provides a reagent comprising a humanized antibody.

The antibody has a Y-shaped structure comprising four polypeptide chains consisting of two identical heavy chains and two identical light chains joined together by disulfide bonds.

The "antibody" used in the present invention is not particularly limited, provided that it can bind specifically to a target antigen, and an isotype thereof may be any of IgG, IgM, IgA, IgE, or IgD. As human immunoglobulins, 9 classes (isotypes) distinguished by 4 subclasses (types), IgG1, IgG2, IgG3, IgG4, IgA1, IgA2, IgD, IgE, and IgM, are known. A range of the IgG antibody defined herein is extensive, and a heavy chain antibody (HCAb) consisting of heavy chains that is observed in animals of Camelidae, such as llama and alpaca is within the scope of the IgG antibody.

IgG is composed of two heavy chains (H chains) and two light chains (L chains). A heavy chain is composed of a variable domain (VH), a first constant domain (CH1), a second constant domain (CH2), and a third constant domain (CH3) arranged in that order from the N terminus. A light chain is composed of a variable domain (VL) and a constant domain (CL) arranged in that order from the N terminus. A region composed of CH2 and CH3 is the Fc region. A heavy chain and a light chain are joined together by a disulfide bond between a cysteine residue in CH1 and a cysteine residue in CL. Heavy chains are joined together by a disulfide bond between cysteine residues in a hinge region between CH1 and CH2.

An antibody may be an IgG fragment comprising a heavy chain, rIgG (reduced IgG) composed of a heavy chain and a light chain, a fragment composed of two heavy chains, or a fragment composed of a heavy chain. An antibody comprising two heavy chains may comprise a sugar chain bound to at least one heavy chain. It is not necessary that sugar chains be bound to both heavy chains. Examples of IgG fragments include Fab, F(ab')2, scFv, Fab', and single-stranded immunoglobulin. An IgG fragment binds to a target antigen, which is a substance to be detected, and it is referred to as a functional fragment of IgG.

### (The humanized antibody of the present invention)

The humanized antibody of the present invention is derived from a non-human animal antibody (typically a mouse antibody) or a chimeric antibody. Such humanized antibody retains or substantially retains antigen-binding properties of the parent antibody but has a weak immunogenic potential in humans. The term "chimeric antibody" used herein refers to an antibody consisting of a variable domain derived from a non-human animal antibody and a constant domain of a human antibody. In the present invention, a "chimeric antibody" is within the scope of the "humanized antibody." In the humanized antibody of the present invention, a heavy chain constant domain is composed of CH1, CH2, and CH3, and a light chain constant domain is the κ or λ constant domain.

The humanized antibody can be potentially produced by: for example, (a) selectively grafting non-human CDRs to the human framework and constant domains (Jones et al., Nature 321: 522-25, 1986; Verhoeyen et al., Science 239: 1534-1536, 1988); or (b) grafting whole non-human variable domains (so as to retain ligand binding properties) and "attaching" a human-like surface thereto by substitution of exposed residues to reduce immunogenicity (also referred to as "veneered antibodies") (Padlan, Molec. Immun., 28: 489-498, 1991; Padlan, Molec. Immun., 31 (3): 169-217, 1994). An antibody produced by the method (a) is referred to as a "CDR-grafted antibody."

The present invention is further characterized in that an antibody is produced by humanizing the Fc region of an antibody. A representative sequence of a heavy chain constant domain constituting the Fc region is as shown below.







 (SEQ ID NO: 1)

The humanized antibody of the present invention may comprise an amino acid sequence other than that of CDR, which is derived from the amino acid sequence of the heavy chain constant domain by substitution, deletion, addition, and/or insertion of a plurality of amino acids, provided that it binds to a target substance. For example, such amino acid sequence may involve substitution, deletion, addition, and/or insertion of 1, 2 or fewer, 3 or fewer, 4 or fewer, 5 or fewer, 6 or fewer, 7 or fewer, 8 or fewer, 9 or fewer, 10 or fewer, 15 or fewer, or 20 or fewer amino acids. The amino acid sequence of the humanized antibody has 90% or higher, 91% or higher, 92% or higher, 93% or higher, 94% or higher, 95% or higher, 96% or higher, 97% or higher, 98% or higher, or 99% or higher identity to the original amino acid sequence free from substitution, deletion, addition, and/or insertion.

The origin of the humanized antibody of the present invention is not limited. For example, the humanized antibody may be derived from any mammal, such as a human or a non-human animal, such as a mouse, rabbit, rat, guinea pig, hamster, goat, sheep, or camel. In addition to a human antibody, a recombinant antibody, such as a humanized antibody or chimeric antibody, can be used. An antibody may be a polyclonal or monoclonal antibody.

The humanized antibody of the present invention can be produced as a recombinant antibody by integrating, for example, DNA encoding a heavy chain variable domain, DNA encoding a heavy chain constant domain, DNA encoding a light chain variable domain, and/or DNA encoding a light chain constant domain into an expression vector, transforming a host cell with the use of the vector, and culturing the host cell. The DNA encoding a heavy chain and the DNA encoding a light chain may be introduced into the same expression vector to transform a host cell with the use of such vector. Alternatively, the DNA encoding a heavy chain and the DNA encoding a light chain may be inserted into different vectors separately from each other to transform a host cell with the use of two vectors. In such a case, DNA may be operably linked to an element, such as a promoter, enhancer, or polyadenylation signal. When DNA is operably linked, DNA is linked to an element to allow the element to exert its function. The vector may comprise DNA encoding a signal peptide that promotes secretion of an antibody from the host cell. After the antibody is produced, a signal peptide can be cleaved, removed, and produced as a mature protein. As a host cell, for example, an animal, bacterial, or yeast cell can be used, and any of known expression vectors for various host cells can be used as an expression vector.

A humanized antibody can be prepared with the use of a plant expression system or with the use of a genetically engineered plant. A conventional transient expression system in plants can be employed. Examples of techniques include, but are not particularly limited to, the agroinfiltration method, the plant virus vector method, the magnICON^{®} system, and the particle gun method.

When the agroinfiltration method is employed, *Agrobacterium* is transformed with the vector, a plant is infected with the *Agrobacterium,* and a plant expressing the protein can be thus obtained.

When the plant expression system is employed, a plant expressing a humanized antibody can be obtained in the manner described below. At the outset, RNA is obtained from cDNA of the plant virus genome comprising DNA encoding a human antibody inserted therein. Subsequently, a plant is inoculated and infected with the RNA vector. Examples of virus vectors include the tobacco mosaic virus (TMV) vector, the plum pox virus (PPV) vector, a potato virus X (PVX) vector, the alfalfa mosaic virus (AIMV) vector, the cucumber mosaic virus (CMV) vector, the cowpea mosaic virus (CPMV) vector, and the zucchini yellow mosaic virus (ZYMV) vector.

When the magnICON^{®} system is employed, a plant expressing a humanized antibody can be obtained in the manner described below. At the outset, cDNA of the TMV or PVX genome comprising DNA encoding a humanized antibody inserted therein is introduced into the T-DNA vector. Subsequently, *Agrobacterium* transformed with the T-DNA vector is allowed to infect a plant.

The produced antibody can be purified by common protein separation and purification techniques. Examples of such techniques include affinity chromatography, ion-exchange chromatography, other chromatography techniques, ultrafiltration, salting out, and dialysis.

The present invention encompasses a reagent used in an immunoassay method, which is a diagnostic or test drug, such as an *in-vitro* diagnostic agent. The reagent may comprise, for example, a buffer to stably retain an antibody. A person skilled in the art can adequately determine other components in accordance with an immunoassay method to be employed.

Examples of immunoassay methods include immunostaining methods (including a fluorescent antibody method, an enzymatic antibody method, a heavy metal-labeled antibody method, and a radioisotope-labeled antibody method), separation by electrophoresis in combination with detection with a fluorescence, enzyme, or radioisotope (including Western blotting and fluorescent two-dimensional electrophoresis), enzyme-linked immunosorbent assay (ELISA), dot blotting, latex agglutination (LA)-turbidimetric immunoassay, and immunochromatography.

Target substances to be detected and quantified with the use of reagents are not particularly limited, provided that Fc-deficient antibodies bind thereto. Target substances may be antigens such as proteins, polysaccharides, monosaccharides, oligosaccharides, amino acids, peptides, hapten including low-molecular-weight physiologically active substances, such as substances having the steroid skeleton, and pathogens, such as bacteria, viruses, and parasites constituted by substances mentioned above. When a target substance is an antigen, examples of target substances include, but are not limited to, protein markers, such as the C-reactive protein (CRP), the prostate-specific antigen, ferritin, β-2 microglobulin, myoglobin, hemoglobin, albumin, and creatinine, immunoglobulins, such as IgG, IgA, and IgM, various tumor markers, lipoproteins, such as LDL,HDL, and TG, virus antigens, such as influenza A virus, influenza B virus, RS virus (RSV), rhinovirus, rotavirus, norovirus, adenovirus, astrovirus, HAV, HBs, HCV, HIV, and EBV, bacterial antigens, such as *Chlamydia trachomatis,* hemolytic *streptococcus, Bordetella pertussis, Helicobacter pylori, leptospire, Treponema pallidum, Toxoplasma gondii, Borrelia, Legionella* bacteria, *Bacillus anthracis,* and MRSA, toxins produced by bacteria, mycoplasma lipid antigens, peptide hormones, such as human chorionic gonadotropin, steroids, such as steroid hormones, bioactive amines, such as epinephrine and morphine, vitamins, prostaglandins, antibiotics, such as tetracycline, agricultural chemicals, and environmental endocrine disruptors. Preferable examples include antigens, such as CRP, the prostate-specific antigen, ferritin, β-2 microglobulin, and hemoglobin.

Other examples of target substances include antibodies that specifically react with the antigens, such as protein markers, various tumor markers, lipoproteins, virus antigens, bacterial antigens, toxins produced by bacteria, peptide hormones, steroids, bioactive amines, vitamins, antibiotics, agricultural chemicals, and environmental endocrine disruptors as mentioned above. Another example of a target substance is the IgE antibody existing *in vivo.* In such a case, antibodies against the aforementioned antibodies may be used as humanized antibodies.

A reagent is added to an analyte derived from a subject in which the presence or absence of a target substance is to be examined. An analyte is not particularly limited, provided that it contains a target substance. Examples thereof include body fluids, such as blood, serum, plasma, urine, feces, saliva, tissue fluid, spinal fluid, and swab specimens, and diluted liquids of any thereof, with blood, serum, plasma, urine, feces, spinal fluid, or diluted liquids of any thereof being preferable.

### (Kit)

The second aspect of the present invention provides a kit comprising the reagent.

The kit can be used for diagnosis, research, and other purposes. The kit may comprise, in addition to an antibody as a reagent, a support to immobilize a humanized antibody thereon, or a secondary antibody, in accordance with the purpose of use. A support is preferably an insoluble support, and examples thereof include latex particles such as polyethylene or polystyrene, alumina particles, silica particles, gold colloids, and magnetic particles. More specifically, a microplate can be used as an insoluble support in ELISA (direct, indirect, sandwich, or other techniques), and latex or the like can be used as an insoluble support in latex aggregation. In order to prevent a non-specific antigen-antibody reaction, the kit preferably comprises a blocking agent.

A humanized antibody may be directly immobilized on a support by physical adsorption or other means, or it may be indirectly immobilized thereon by chemical crosslinking, such as via a linker.

A kit used in the direct method comprises, as a reagent containing a humanized antibody, for example, an enzyme-labeled antibody binding to an antigen, a substrate used for an enzyme reaction, and a standardized antigen used for preparation of a calibration curve or a control experiment.

A kit used in the indirect method comprises, as a reagent containing a humanized antibody, for example, a primary antibody binding to an antigen, a secondary antibody binding to the primary antibody, a substrate used for an enzyme reaction, and a standardized antigen used for preparation of a calibration curve or a control experiment. The secondary antibody may be labeled for detection.

A kit used in the sandwich method comprises, as a reagent containing a humanized antibody, for example, an antibody immobilized on a solid phase, an enzyme-labeled antibody, a substrate used for an enzyme reaction, and a standardized antigen used for preparation of a calibration curve or a control experiment. The enzyme-labeled antibody may be a humanized antibody.

A kit used in latex agglutination comprises, as a reagent containing a humanized antibody, for example, a binding antibody for detection, a standardized antigen used for preparation of a calibration curve or a control experiment, and a blocking agent for preventing a non-specific antigen-antibody reaction.

### (Method of measurement)

The third aspect of the present invention provides a method for measuring a target substance with the use of an antigen-antibody reaction using a humanized antibody.

The method can be performed with the use of the reagent or kit, and the method is performed by the immunological method.

### (Method for inhibiting non-specific reaction)

The fourth aspect of the present invention provides a method for inhibiting a non-specific antigen-antibody reaction using a humanized antibody.

Use of a humanized antibody enables inhibition of a non-specific reaction mediated by a non-specific reactive factor, such as a complement (C1q), an Fc receptor, a rheumatoid factor, or a heterophilic antibody. A non-specific reaction mediated by an Fc receptor occurs when an analyte contains a receptor directed against the Fc region of the antibody . A non-specific reaction mediated by a rheumatoid factor is caused by an antibody directed against the Fc region of the antibody. A non-specific reaction mediated by a heterophilic antibody is caused by an antibody directed against a non-human animal antibody existing in human blood. A non-specific reaction may be caused when a substance unrelated to the antigen-antibody reaction of interest generates an antigen-antibody reaction product or shows cross reactivity. Accordingly, a humanized antibody is used preferably in combination with a method for inhibiting a non-specific reaction known to a person skilled in the art.

The method for inhibiting a non-specific reaction comprises a step of bringing a humanized antibody into contact with an analyte. The method may comprise any step before or after the step of contact.

With the use of a humanized antibody, a non-specific antigen-antibody reaction can be inhibited, and a target substance can be measured accurately.

### Examples

The present invention is described in greater detail with reference to the following examples, although the present invention is not limited to these examples.

### <Preparation of anti-C-reactive protein (CRP) humanized antibody (expressed in plant)>

### 1. Preparation of vector and expression of protein

As an antibody with the humanized Fc region, a mouse-derived humanized anti-CRP monoclonal antibody (IgG) was selected.

The IgG heavy chain was cloned into the tobacco mosaic virus (TMV) vector (Icon Genetics), and the IgG light chain was cloned into the potato virus X (PVX) vector (Icon Genetics). These vectors were transformed into separate *Agrobacterium* cells, and the transformed cells were then cultured. Thereafter, the mixed cultures were co-infiltrated into *Nicotiana benthamiana* leaves, the leaves were harvested approximately 1 week later, and the harvested leaves (infected leaves) were then frozen.

### 2. Purification

The frozen infected leaves were ground using a cutter mixer, a buffer (100 mM Tris, 250 mM NaCl, 5 mM EDTA, 40 mM sodium ascorbate) was added in equal amount by weight, and the infected leaves were further ground. The solution containing the ground infected leaves was collected from the cutter mixer, and the collected solution was centrifuged at 5°C and 15,000× g for 30 minutes to collect a supernatant. The supernatant was filtered through a 0.45 µm filter and subjected to protein A column chromatography (MabSelect, SuRe pcc, Global Science & Technology). Thus, plant-derived impurities and antibody-derived impurities were separated and removed.

### 3. Expression test

An extraction buffer (100 mM Tris, 250 mM NaCl, 5 mM EDTA) was added to the infected leaves in an amount equal to 3 times the amount of the infected leaves. The infected leaves were ground using a bead mill, allowed to stand on ice for 30 minutes, and centrifuged at 8,000× g and 4°C for 10 minutes to collect a supernatant. The supernatant was then analyzed by SDS-PAGE. As a result, expression of a mouse-derived humanized anti-CRP antibody as the antibody with the humanized Fc region was observed.

### <Preparation of anti-C-reactive protein (CRP) humanized antibody (expressed in CHO cell)>

### 1. Preparation of vector and expression of protein

As an antibody with the humanized Fc region, a mouse-derived humanized anti-CRP monoclonal antibody (IgG) was selected.

The IgG heavy chain and the IgG light chain were cloned into the pCHO1.0 vector (Thermo Fisher Scientific). The vector was linearized with restriction enzymes and then transfected into Freedom^{™} CHO-S^{™} (Thermo Fisher Scientific). Thereafter, clones expressed in stable CHO producing mouse-derived humanized anti-CRP monoclonal antibodies were obtained in accordance with the Freedom^{™} CHO-S^{™} Kit User Guide (Thermo Fisher Scientific). The clones were fed-batch cultured for 14 days, and a solution obtained by removing cells from the culture solution was collected.

### 2. Purification

The solution from which cells had been removed was centrifuged at 4°C and 15,000× g for 10 minutes to collect a supernatant. The supernatant was filtered through a 0.22 µm filter and subjected to protein A column chromatography (HiTrap^{™} MabSelect SuRe^{™}, Global Science & Technology). Thus, CHO-cell-derived impurities were separated and removed.

### 3. Expression test

The solution from which cells had been removed was filtered through a 0.22 µm filter and then analyzed by SDS-PAGE.

As a result, expression of a mouse-derived humanized anti-CRP antibody as the antibody with the humanized Fc region was observed.

### <Measurement of CRP-negative human serum by the method performed with the addition of a compound that inhibits a non-specific reaction caused by a heterophilic antibody>

### (1) Preparation of reagent

With the use of an antibody against CRP, an assay reagent for immunoagglutination was prepared in the manner described below.
i) Sensitized particles comprising the rabbit-derived anti-CRP polyclonal antibody supported thereon in an amount of 0.26 mg relative to 1 ml of a polystyrene latex suspension was suspended in a buffer (glycine, pH 7.3) to 0.18 (w/v)% to prepare a latex suspension.
ii) A buffer (glycine, pH 7.0) was prepared.

### (2) Preparation of standard solution

With the use of CRP, a standard solution used for preparing a calibration curve was prepared.

### (3) Measurement using automatic analyzer

Measurement was performed automatically by an end-point assay using the Hitachi 7180 automatic analyzer.

With the use of the aforementioned reagent, physiological saline and 3 CRP-negative human serum analytes (commercially available from Golden West Biologicals) were measured. To 2.5 µl of the analyte solution, 125 µl of a buffer (glycine, pH 7.0) was added, and the resulting mixture was agitated at 37°C. After the mixture was allowed to stand for 5 minutes, 125 µl of the latex suspension was added, and the mixture was further agitated at 37°C. The results of the agglutination reaction performed for approximately 3 minutes were measured as the changes in absorbance, and the CRP concentration of each analyte was determined based on the calibration curve prepared with the use of the standard solution (Table 1, Figure 1).

**[Table 1]**

| Analyte | CRP concentration (mg/ml) |
|---|---|
| Physiological saline | 0.00 |
| CRP-negative human serum (1) | 0.00 |
| CRP-negative human serum (2) | 0.00 |
| CRP-negative human serum (3) | 0.00 |

### <Measurement of CRP-negative human serum by the method performed without the addition of a compound that inhibits a non-specific reaction caused by a heterophilic antibody>

### (1) Preparation of reagent

With the use of an antibody against CRP, an assay reagent for immunoagglutination was prepared in the manner described below.
i) Sensitized particles comprising the rabbit-derived anti-CRP polyclonal antibody supported thereon in an amount of 0.26 mg relative to 1 ml of a polystyrene latex suspension was suspended in a buffer (glycine, pH 7.3) to 0.18 (w/v)% to prepare a latex suspension.
ii) A latex reagent of the "N-assay LA CRP-T Nittobo" (Nittobo Medical Co., Ltd.) using the goat-derived anti-CRP polyclonal antibody (anti-human CRP goat polyclonal antibody-sensitized latex particles) was prepared.
iii) Sensitized particles comprising the mouse-derived humanized anti-CRP antibody (expressed in plants) supported thereon in an amount of 0.11 mg relative to 1 ml of a polystyrene latex suspension was suspended in a buffer (glycine, pH 7.3) to 0.15 (w/v)% to prepare a latex suspension.
iv) Sensitized particles comprising the mouse-derived humanized anti-CRP antibody (expressed in CHO cell) supported thereon in an amount of 0.11 mg relative to 1 ml of a polystyrene latex suspension was suspended in a buffer (glycine, pH 7.3) to 0.16 (w/v)% to prepare a latex suspension.
v) A buffer (phosphate, pH 7.4) was prepared.

### (2) Measurement using automatic analyzer

Measurement was performed automatically by an end-point assay using the Hitachi 7180 automatic analyzer.

With the use of the aforementioned reagent, physiological saline and 3 CRP-negative human serum analytes (commercially available from Golden West Biologicals) were measured. To 3.0 µl of the analyte solution, 240 µl of a buffer (phosphate, pH 7.4) was added, and the resulting mixture was agitated at 37°C. After the mixture was allowed to stand for 5 minutes, 36 µl of the latex suspension was added, and the mixture was further agitated at 37°C. The results of the agglutination reaction performed for approximately 90 seconds were measured as the changes in absorbance (Figure 1).

As shown in Figure 1, the analyte that was measured to have the CRP measurement of 0.00 mg/ml by the method performed with the addition of a compound that inhibits a non-specific reaction caused by a heterophilic antibody was measured by the method performed without the addition of a compound that inhibits a non-specific reaction caused by a heterophilic antibody. As a result, significant changes in absorbance were measured with the use of reagent comprising the rabbit-derived anti-CRP antibody or the goat-derived anti-CRP antibody, compared with physiological saline. In contrast, the changes in absorbance similar to those observed in physiological saline were measured with the use of the mouse-derived humanized anti-CRP antibody. If the CRP measurement is 0.00 mg/ml, the changes in absorbance are considered to be similar to those observed in physiological saline. Accordingly, a non-specific agglutination is determined to have occurred when measuring analytes with the use of the reagent comprising the rabbit-derived anti-CRP antibody. In contrast, the changes in absorbance similar to those observed in physiological saline were measured with the use of the mouse-derived humanized anti-CRP antibody. Accordingly, reagent comprising the mouse-derived humanized anti-CRP antibody or the goat-derived anti-CRP antibody is considered to less likely to cause a non-specific agglutination when measuring analytes by the method performed without the addition of a compound that inhibits a non-specific reaction caused by a heterophilic antibody.

### Industrial Applicability

The method of the present invention enables accurate measurement of a target substance in human blood.

All publications, patents, and patent applications cited herein are incorporated herein by reference in their entirety.

## Claims

1. An immunoassay reagent comprising a humanized antibody against a target substance.

2. The reagent according to claim 1, wherein the humanized antibody inhibits a non-specific reaction.

3. The reagent according to claim 1 or 2, wherein the humanized antibody is IgG.

4. The reagent according to claim 1 or 2, wherein the humanized antibody is produced using a plant expression system.

5. The reagent according to any one of claims 1 to 4, which is used for diagnosis.

6. The reagent according to any one of claims 1 to 5, which is used for research.

7. A kit comprising the reagent according to any one of claims 1 to 6.

8. An immunoassay method comprising bringing a humanized antibody against a target substance into contact with the target substance.

9. The immunoassay method according to claim 8, wherein the humanized antibody inhibits a non-specific reaction.

10. The immunoassay method according to claim 8 or 9, wherein the humanized antibody is IgG.

11. The immunoassay method according to claim 8 or 9, wherein the humanized antibody is produced using a plant expression system.

12. A method for inhibiting a non-specific antigen-antibody reaction in an immunoassay method involving the use of a humanized antibody against a target substance.

13. The method for inhibiting a non-specific reaction according to claim 12, wherein the humanized antibody inhibits a non-specific reaction.

14. The method for inhibiting a non-specific reaction according to claim 12 or 13, wherein the humanized antibody is IgG.

15. The method for inhibiting a non-specific reaction according to claim 12 or 13, wherein the humanized antibody is produced using a plant expression system.
